# EUROPÄISCHE PATENTSCHRIFT

(11) **EP 0 499 930 B1**
(45) Veröffentlichungstag und Bekanntmachung des Hinweises auf die Patenterteilung: **01.07.1998**
(21) Anmeldenummer: 92102160.6
(22) Anmeldetag: 10.02.1992
(51) Int. Cl.: C07D 239/30

(54) **Verfahren zur Herstellung von 5-Fluorpyrimidinen**
Process for the preparation of 5-fluoropyrimidines
Procédé pour la préparation de 5-fluoropyrimidines

(30) Priorität: 22.02.1991 DE 4105553
(43) Veröffentlichungstag der Anmeldung: 26.08.1992
(73) Patentinhaber: BAYER AG, 51368 Leverkusen (DE)
(72) Erfinder: Bielefeldt, Dietmar, Dr., W-4030 Ratingen 6 (DE); Braden, Rudolf, Dr., W-5068 Odenthal (DE)

(56) Entgegenhaltungen:
- CH-A- 647 512
- US-A- 4 786 733
- JOURNAL OF FLUORINE CHEMISTRY Bd. 25, 1984, Seiten 435 - 446; M.M. BOUDAKIAN ET AL: 'Substitutive aromatic fluorination with chlorine pentafluoride'

## Beschreibung

Die vorliegende Erfindung betrifft ein besonders vorteilhaftes Verfahren zur Herstellung von 5-Fluorpyrimidinen.

Es ist bekannt, daß man 5-Fluorpyrimidine herstellen kann, indem man 2,4,6-Trifluorpyrimidin mit ClF₅ umsetzt (siehe J. Fluorine Chem. 25, 435 (1984)). Nachteilig bei diesem Verfahren ist, daß man 5-Fluorpyrimidine stets im Gemisch mit 5-Chlorpyrimidinen erhält, die Ausbeute an 5-Fluorpyrimidinen niedrig und ClF₅ wegen besonders stark ausgeprägten korrosiven Eigenschaften schwierig zu handhaben ist.

Es ist ferner bekannt 5-Fluor-trichlorpyrimidin aus Tetrafluorpyrimidin durch Rückchlorierung mit HCl im Autoklaven herzustellen (siehe J. Fluorine Chem. 45, 417 (1989)). Auch hier sind die Ausbeuten niedrig. Die Arbeitsweise mit HCl unter Druck erfordert besonderen Aufwand.

Ferner wird in Kirk-Othmer, Encyclopedia of Chemical Technology Band 10, Seite 901 (1980) auf die Heftigkeit der Reaktion bei der aromatischen Substitution mit molekularem Fluor hingewiesen und indirekte Fluorierungen und nicht näher bezeichnete neue Fluorierungsmittel empfohlen.

CH-A 647 512 beschreibt die Herstellung von 2,4,5-Trifluor-pyridin aus entsprechenden Chlor- und/oder Bromverbindungen mit einem Metallfluorid.

Gemäß der US-A 4 786 733 wurden substituierte Pyridine, insbesondere alkylsubstituierte Pyridine direkt zu den entsprechenden 2-Fluor-alkylsubstituierten Pyridinen fluoriert.

Es wurde nun ein Verfahren zur Herstellung von 5-Fluorpyrimidinen der Formel (I) in der 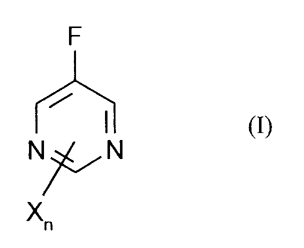
X unabhängig voneinander für Fluor oder Chlor und
n für 1, 2 oder 3 stehen, gefunden,
das dadurch gekennzeichnet ist, daß man ein halogeniertes Pyrimidin der Formel (II) in der 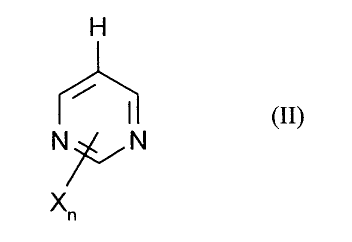
X und n die bei Formel (I) angegebene Bedeutung haben,
in Gegenwart von Trichlorfluormethan, Dichlordifluormethan, Perfluorhexan, Perfluoroctan, Trifluoressigsäure und/oder einem Vakuumpumpenöl auf der Basis von Perfluorpolyethern (Fomblin®) und in Gegenwart von Natriumfluorid, Kaliumfluorid oder basischem Aluminiumoxid mit elementarem Fluor in Form eines Fluor/Inertgas-Gemisches, das 0,5 bis 50 Gew.-%

Fluor enthält, umsetzt und dabei Temperaturen im Bereich -100 bis +80 C und Drucke im Bereich 0,1 bis 5 bar einhält.

Wenn nur Fluor enthaltende Pyrimidine der Formel (II) eingesetzt werden, erhält man direkt reine 5-Fluorpyrimidine der Formel (I). Wenn man Chlor enthaltende Pyrimidine der Formel (II) einsetzt, erhält man im allgemeinen Gemische von 5-Fluorpyrimidinen der Formel (I), die Chlor und gegebenenfalls weiteres Fluor enthalten.

Pro Mol des eingesetzten halogenierten Pyrimidins der Formel (II) kann man beispielsweise 0,2 bis 5 mol elementares Fluor einsetzen. Die Konzentration an Fluor in dem Fluor/Inertgas-Gemisch kann beispielsweise 5 bis 50 Gew.-% betragen.

Als Lösungsmittel für das das erfindungsgemäße Verfahren ist Trichlorfluormethan bevorzugt.

Die als Ausgangsprodukte benötigten halogenierten Pyrimidine der Formel (II) sind Handelsprodukte der Fa. Aldrich oder gemäß US-A-3314955 erhältlich.

Das nach der Reaktion vorliegende Gemisch kann man beispielsweise aufarbeiten, indem man den an Natriumfluorid, Kaliumfluorid oder basisches Aluminiumoxid gebundenen Fluorwasserstoff filtriert und das Filtrat einer Destillation, gegebenenfalls unter vermindertem Druck, unterwirft.

Das erfindungsgemäße Verfahren wird vorzugsweise bei Normaldruck durchgeführt.

Insbesondere perhalogenierte 5-Fluorpyrimidine der Formel (I), d.h. solche, bei denen n für 3 steht, lassen sich auf die erfindungsgemäße Weise besonders gut und einfach herstellen.

Mit Hilfe des erfindungsgemäßen Verfahrens gelingt es, 5-Fluorpyrimidine der Formel (I) in guten Ausbeuten, bei niederen Temperaturen und ohne Druckanwendung herzustellen.

5-Fluorpyrimidine der Formel (I) sind wichtige Zwischenprodukte, beispielsweise zur Herstellung von pharmakologisch wirksamen Substanzen. Man kann pharmakologisch wirksame Mittel z.B. herstellen, indem man ein 5-Fluorpyrimidinderivat der Formel (I) katalytisch hydriert und anschließend hydrolysiert (siehe J. Fluorine Chem. 45, 417 (1989)).

### Beispiele

### Beispiel 1

In eine Suspension aus 29,0 g (0,216 mol) 2,4,6-Trifluorpyrimidin, 21 g Natriumfluorid (0,5 mol) und 250 ml Trichlorfluormethan wurden bei einer Temperatur von -78°C 0,5 mol Fluor in Form einer 30 vol.-%igen Mischung mit Helium eingeleitet. Die Einleitungsgeschwindigkeit betrug 0,15 mol Fluor pro Stunde. Nach Beendigung der Einleitung wurde das Reaktionsgemisch filtriert und aus dem Filtrat 21,0 g Tetrafluorpyrimidin (= 64 % der Theorie) durch Destillation erhalten.

### Beispiel 2

In eine Suspension aus 36,7 g (0,2 mol) 2,4,6-Trichlorpyrimidin, 21 g Natriumfluorid (0,5 mol) und 250 ml Trichlorfluormethan wurden bei einer Temperatur von -78°C 0,5 mol Fluor als 30 vol.-%ige Mischung mit Helium eingeleitet. Die Einleitungsgeschwindigkeit lag bei 0,15 mol Fluor pro Stunde. Nach Beendigung des Einleitens wurde das Reaktionsgemisch abfiltriert und aus dem Filtrat durch Destillation über Eisenspäne 19,3 g reines 5-Fluor-2,4,6-trichlorpyrimidin erhalten. Das entspricht einer Ausbeute von 48 % der Theorie. Der Siedepunkt des isolierten Produktes betrug 80-87°C bei 10 mbar. Daneben enthielt das Reaktionsgemisch noch höher fluorierte Pyrimidinderivate.

### Beispiel 3

In eine Suspension aus 92 g (0,5 mol) 2,4,6-Trichlorpyrimidin, 100 g Natriumfluorid und 1000 ml Trichlorfluormethan wurden bei -78°C 0,55 mol Fluor als 18 vol.-%ige Mischung mit Helium eingeleitet. Nach Beendigung der Einleitung wurde das Reaktionsgemisch filtriert und aus dem Filtrat 59,4 g (= 59 % der Theorie) 5-Fluor-2,4,6-trichlorpyrimidin abdestilliert.

## Patentansprüche

1. Verfahren zur Herstellung von 5-Fluorpyridinen der Formel (I) in der 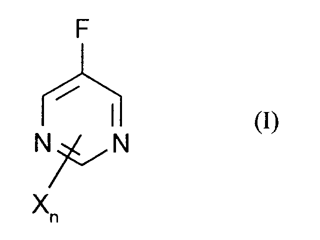
X unabhängig voneinander für Fluor oder Chlor und
n für 1, 2 oder 3 stehen,
das dadurch gekennzeichnet ist, daß man ein halogeniertes Pyrimidin der Formel (II) in der 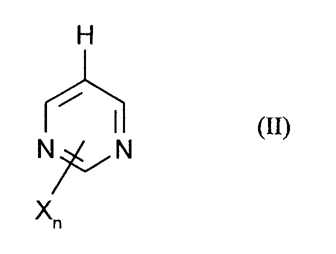
X und n die bei Formel (I) angegebene Bedeutung haben,
in Gegenwart von Trichlorfluormethan, Dichlordifluormethan, Perfluorhexan, Perfluoroctan, Trifluoressigsäure und/oder einem Vakuumpumpenöl auf der Basis von Perfluorpolyethern (Fomblin®) und in Gegenwart von Natriumfluorid, Kaliumfluorid oder basischem Aluminiumoxid mit elementarem Fluor in Form eines Fluor/Inertgas-Gemisches, das 0,5 bis 50 Gew.-% Fluor enthält, umsetzt und dabei Temperaturen im Bereich -100 bis +80 C und Drucke im Bereich 0,1 bis 5 bar einhält.

2. Verfahren nach Anspruch 1, dadurch gekennzeichnet, daß man pro mol des eingesetzten halogenierten Pyrimidins der Formel (II) 0,5 bis 3 mol elementares Fluor einsetzt.

3. Verfahren nach Ansprüchen 1 bis 2, dadurch gekennzeichnet, daß man es bei Normaldruck durchführt.

4. Verfahren nach Ansprüchen 1 bis 3, dadurch gekennzeichnet, daß man das nach der Reaktion vorliegende Gemisch aufarbeitet, indem man den an die Base gebundendem Fluorwasserstoff, filtriert und das Filtrat einer Destillation unterwirft.

## Claims

1. Process for the preparation of 5-fluoropyridines of the formula (I) 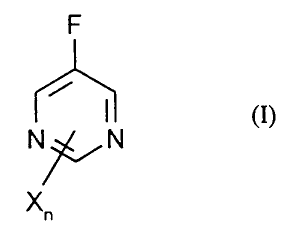 in which
X represents, independently of each other, fluorine or chlorine and
n is 1, 2 or 3,
which is characterised in that a halogenated pyrimidine of the formula (II) 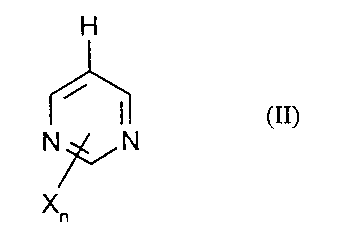 in which
X and n have the meanings defined as for formula (I),
is reacted with elemental fluorine in the form of a fluorine/inert gas mixture containing 0.5 to 50% by weight of fluorine, in the presence of trichlorofluoromethane, dichlorodifluoromethane, perfluorohexane, perfluorooctane, trifluoroacetic acid and/or a vacuum pump oil based on perfluoropolyethers (Fomblin®) and in the presence of sodium fluoride, potassium fluoride or basic aluminium oxide, and during the reaction temperatures are maintained in the range -100 to +80°C and pressures in the range of 0.1 to 5 bar.

2. Process according to Claim 1, characterised in that 0.5 to 3 mol of elemental fluorine is used per mole of the halogenated pyrimidine of the formula (II) used.

3. Process according to Claims 1 to 2, characterised in that atmospheric pressure is employed.

4. Process according to Claims 1 to 3, characterised in that the mixture present after the reaction is worked up by filtering the hydrogen fluoride bound to the base, and subjecting the filtrate to a distillation.

## Revendications

1. procédé de préparation de 5-fluoropyrimidines de formule (I) 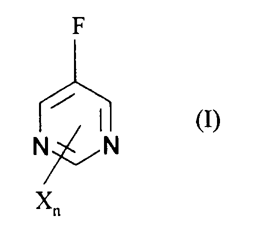 où
les X représentent indépendamment les uns des autres le fluor ou le chlore et
n représente 1, 2 ou 3,
qui est caractérisé en ce que l'on fait réagir une pyrimidine halogénée de formule (II) 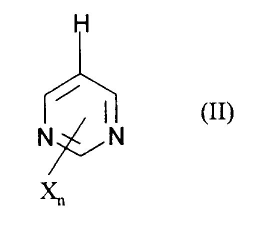 où
X et n ont la signification indiquée au sujet de la formule (I),
en présence de trichlorofluorométhane, de dichlorodifluorométhane, de perfluorohexane, de perfluorooctane, d'acide trifluoroacétique et/ou d'une huile de pompe à vide à base de perfluoropolyéthers (Fomblin®) et en présence de fluorure de sodium, de fluorure de potassium ou d'oxyde d'aluminium basique, avec du fluor élémentaire sous forme d'un mélange fluor/gaz inerte qui contient 0,5 à 50 % en masse de fluor, en maintenant des températures dans le domaine de -100 à +80°C et des pressions dans le domaine de 0,1 à 5 bar.

2. Procédé selon la revendication 1, caractérisé en ce que l'on utilise par mole de pyrimidine halogénée de formule (II) utilisée 0,5 à 3 mol de fluor élémentaire.

3. Procédé selon les revendications 1 et 2, caractérisé en ce qu'on le met en oeuvre à la pression normale.

4. Procédé selon les revendications 1 à 3, caractérisé en ce que l'on traite le mélange existant après la réaction en filtrant le fluorure d'hydrogène lié à la base et en soumettant le filtrat à une distillation.
